(19) Europäisches Patentamt

European Patent Office

Office européen des brevets

(11) EP 0 510 276 B1

(12) **EUROPEAN PATENT SPECIFICATION**

(45) Date of publication and mention
of the grant of the patent:
**11.09.1996 Bulletin 1996/37**

(51) Int. Cl.$^6$: **A61L 11/00**, B02C 23/40,
B02C 21/00

(21) Application number: **91305025.8**

(22) Date of filing: **03.06.1991**

(54) **Multi-stage infectious waste treatment system**

Mehrstufiges Behandlungssystem für infektiösen Müll

Système de traitement de déchets infectieux en plusieurs étapes

(84) Designated Contracting States:
**DE FR GB IT NL**

(30) Priority: **23.04.1991 US 690116**

(43) Date of publication of application:
**28.10.1992 Bulletin 1992/44**

(73) Proprietor: **WINFIELD INDUSTRIES
San Diego, California 92121 (US)**

(72) Inventor: **Meijer, Robert S.
San Diego, California 92123 (US)**

(74) Representative: **Coxon, Philip et al
Eric Potter & Clarkson
St. Mary's Court
St. Mary's Gate
Nottingham NG1 1LE (GB)**

(56) References cited:
**EP-A- 0 383 553          WO-A-90/03949
DE-A- 2 512 024          US-A- 4 578 185
US-A- 4 884 756**

## Description

### FIELD OF THE INVENTION

The present invention relates generally to treatment of infectious waste. More particularly, the present invention relates to a system which mechanically fragments and decontaminates infectious waste. The present invention is particularly, though not exclusively, a system for mechanically fragmenting infectious waste with a series of blades while simultaneously treating the waste with a disinfectant that is formulated within the system.

### BACKGROUND OF THE INVENTION

The disposal of infectious waste from hospitals and other medical establishments is a major problem. Indeed, the importance of proper and effective infectious waste disposal has become of greater concern in recent years, due to an increased awareness of health problems such as the AIDS epidemic. In part because of the AIDS epidemic, definitions of what constitutes "infectious waste" are being broadened. Consequently, the volume of infectious waste which must be disposed of is increasing. Accordingly, the need for a system or apparatus which will accomplish the safe, efficacious, and cost effective treatment of significant volumes of infectious waste for disposal is growing.

One method for decontaminating infectious waste involves incineration, wherein the waste is burned and the decontaminated ashes are properly disposed. An alternative treatment method is to disinfect the waste in a steam autoclave or ethylene oxide autoclave prior to waste disposal. While effective for their intended purposes, both incinerators and autoclaves present ancillary problems. Incinerators, for example, are difficult and costly to construct and are relatively expensive to maintain in an environmentally safe manner. Autoclaves too, present additional problems, such as odor, cost and operational complexity. Additionally, waste which has been disinfected by autoclaving typically requires further treatment procedures, such as incineration, prior to final disposition of the waste in such places as landfills.

With the above discussion in mind, alternative infectious waste treatment systems have been proposed to disinfect the waste in preparation for disposal. According to these proposals, a solid infectious waste is contacted with a disinfectant solution containing a chlorine compound to decontaminate the waste. The decontaminated waste may then be disposed in ordinary landfills.

Unfortunately, decontamination of waste using chlorine compounds presents certain technical complications. First, liquid disinfectant loses its disinfectant potency during prolonged storage. Thus, there is a need to use liquid disinfectant that is relatively "fresh" in order to achieve an acceptable degree of waste decontamination. Second, it is relatively difficult to ensure that an appropriate concentration of the disinfectant has been contacted with the waste during the treatment process. It is also important, however, to avoid applying too high a concentration of chlorine compound to the waste, in order to avoid undesirable results, such as corrosive effects and the release of toxic gasses. The present invention recognizes that precise amounts of dry disinfectant precursors can be stored for relatively lengthy time periods without losing their potency and can be mixed with water to form a chlorinated disinfectant solution when needed. The resulting solution can be used to decontaminate infectious waste in a system that mechanically shreds the waste.

US 4 884 756 discloses apparatus for treating waste having the features of the precharacterizing portion of claim 1

Accordingly, it is an object of the present invention to provide a system for waste treatment in which precise amounts of a chlorine-based disinfectant are blended with infectious waste to decontaminate the waste. Another object of the present invention is to provide a system for waste treatment which results in particle size reduction, and correspondingly bulk volume of the infectious waste while it is being disinfected. Finally, it is an object of the present invention to provide a system for waste treatment which is relatively easy and comparatively cost-effective to implement.

### SUMMARY OF THE INVENTION

The invention provides a multi-stage system for treating an infectious waste along a continuous flowpath comprising: an inlet stage having an opening sized to receive an infectious waste into the flowpath of said system; a shredding stage for reducing the particle size of the waste to a small particle size; a disinfecting stage comprising a reactor chamber positioned to receive the wetted granular waste and sized to retain the waste for a selected residence time; an auger to transport the waste to a waste discharge port; and a dewatering stage; characterized in that the shredding stage comprises a plurality of opposingly rotatable blades in the flowpath of the waste; and further comprising a wetting stage comprising a liquid jet positioned at the flowpath to inject a liquid medium into the waste and to simultaneously wet and mix a disinfectant in the waste material; a granulating stage provided beneath the wetting stage for further reducing the particle size of the waste to a smaller granular particle size, said granulating stage having at least one stationary blade and at least one rotatable blade rotatably positionable against said stationary blade to form a cutting surface; and wherein the dewatering stage comprises a flow restriction in the waste flowpath, said dewatering stage being in fluid communication with said wetting stage to recycle water from said dewatering stage to said wetting stage.

A preferred embodiment of the present invention provides a system for treating infectious waste comprising a series of continuous treatment stages. The multi-stage treatment system has an inlet stage as its front end which comprises an opening for receiving the infectious waste and desired treatment chemicals. The waste may be fed in any form through the opening, but in a preferred embodiment, the opening is sized to receive a sealed compartmentalized plastic bag in which the waste and treatment chemicals are stored. The bags are fed through the opening into the system in their entirety. In this manner, waste handlers operating the present system need never come in direct contact with the infectious waste or treatment chemicals. The preferred waste bag has a primary compartment containing the infectious waste, and has one or more secondary prefilled and sealed compartments containing the disinfectant chemicals or other process additives in isolation from the waste all of which are to be introduced into the system. As will be seen below, the entire contents of the bag are released from the bag and commingled during operation of the treatment system.

The inlet opening accesses a fragmenting chamber positioned therebelow which encloses the shredding, wetting and granulating stages of the system. The waste drops under the force of gravity from the inlet stage down into the shredding stage which comprises a plurality of opposingly rotating blades. The shredding blades destroy the waste bag, spilling its contents into the fragmenting chamber. Any disinfectant chemicals or process additives contained in the bag become mixed with the waste in the shredding stage. The blades also function to break up the large frangible waste into small size particles.

The wetting stage is performed in conjunction with the shredding stage to wet the mixture of small particle size waste and disinfectant chemicals or additives as they fall through the shredding blades. The wetting stage comprises a plurality of jets positioned at the interior walls of the fragmenting chamber adjacent the shredding blades. The jets are directed radially into the chamber and are capable of producing a controlled spray of a liquid medium into the waste mixture. The liquid medium is preferably an aqueous liquid, such as fresh tap water and/or recycled process water, which has been preheated to an elevated temperature and which uniformly contacts the falling waste mixture to form a hot mash.

The granulating stage is provided beneath the wetting stage and comprises a plurality of travelling blades axially mounted on a shaft and rotatable against a pair of stationary blades mounted on the walls of the fragmenting chamber to form cutting surfaces. The traveling blades rotate in a radial plane which is substantially parallel to the flow of the mash. At the cutting surfaces, the granulating blades break up the already small particle size waste into yet smaller particle sizes and cut any fibrous material which has not been previously fragmented by the shredding blades. The blades also more fully mix the components of the waste mash, thereby dissolving at least a portion of the disinfectant chemicals in the liquid medium to form a disinfectant solution.

The granulating stage product preferably is fully wetted by the disinfectant solution and in gross has a smaller granular particle size than the product of the shredding stage.

The outlet conduit from the fragmenting chamber houses the screening stage which comprises a screen functioning in cooperation with the granulating stage. The screen is sized to allow the smaller granular particle size waste to fall through the screen into a disinfectant reactor chamber below while retaining any waste which has not been sufficiently fragmented in the granulating stage. Waste which is retained by the screen is scooped up by the traveling blades rotating against the screen and returned to the cutting surfaces for additional particle size reduction until the waste is sufficiently small to pass through the screen. It is noteworthy that up to this point substantially all of the work to convey the waste through the above-recited stages is performed by gravity.

The screening stage is followed by the disinfecting stage. The disinfecting stage comprises a disinfectant reactor chamber preferably integral with an auger. The auger has two ends; a liquid medium collection tank and inlet port are at one end of the auger and a disinfected solid waste discharge port is at the other end. The auger is inclined upwardly toward the disinfected solid waste discharge port and accordingly the auger conveys the waste upwardly from the inlet port to the discharge port The length of the auger wherein the disinfection reaction occurs constitutes the disinfectant reactor chamber. The disinfection reaction is preferably completed by the time the waste reaches a point about two-thirds up the auger incline. The controlled rate at which the auger screw carries the waste up the incline to the discharge port enables a sufficient residence time for disinfection of the waste.

The final stage is the dewatering stage. The dewatering stage comprises a flow restriction in the flowpath of the waste. Although some of the liquid medium is removed from the waste by gravity at the lower end of the auger, the bulk of the liquid medium is removed from the waste by compressing the mash through the flow restriction which is preferably positioned adjacent the waste discharge port. The liquid medium driven from the mash at the flow restriction exits the auger through perforations in the housing and is passed to the heated collection tank for recycling to the wetting stage.

In operation, process control for the present system is provided by regulating the disinfectant concentration in the system as a function of the liquid medium temperature. Temperature is in turn a function of liquid medium flow, and heater and auger operating parameters. It is apparent that the above-described system satisfies the present objective of providing an infectious waste treatment apparatus which contacts precise amounts of a disinfectant with an infectious waste to disinfect the waste while simultaneously fragmenting the waste to reduce its bulk volume. It is further apparent that the system provides an infectious waste disposal apparatus which is relatively easy and comparatively cost-effective to implement and operate.

The novel features of this invention, as well as the invention itself, both as to its structure and its operation, will be best understood from the accompanying drawings, taken in conjunction with the accompanying description, in which similar reference characters refer to similar parts, and in which:

BRIEF DESCRIPTION OF THE DRAWINGS

Figure 1 is a perspective of the multi-stage waste treatment system of the present invention;

Figure 2 is a cross-section of the multi-stage waste treatment system of the present invention along line 2-2;

Figure 3 is a cross-section of the multi-stage waste treatment system of the present invention along line 3-3;

Figure 4 is a cross-section of another embodiment of the waste outlet flow restriction; and

Figure 5 is a schematic of a control unit for the multi-stage waste treatment system of the present invention.

Figure 6 is a generalized curve for the functional relation between disinfectant solution temperature and disinfectant concentration.

DESCRIPTION OF PREFERRED EMBODIMENTS

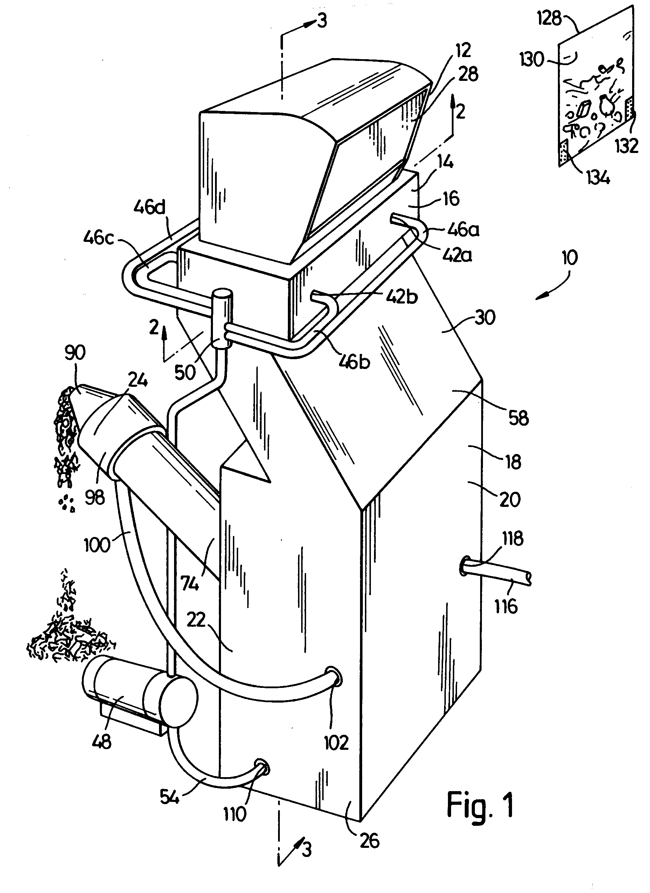

Referring initially to Figure 1, the infectious waste treatment system of the present invention is generally designated **10**. System **10** comprises a plurality of treatment stages including an inlet stage **12**, a shredding stage **14**, a wetting stage **16**, a granulating stage **18**, a screening stage **20**, a disinfecting stage **22**, and a dewatering stage **24** which define a continuous flowpath for the waste. The terms "disinfect" and "decontaminate" are used synonymously herein and refer to the destruction of a substantial portion of infectious constituents within the infectious waste sufficient to render the waste substantially noninfectious.

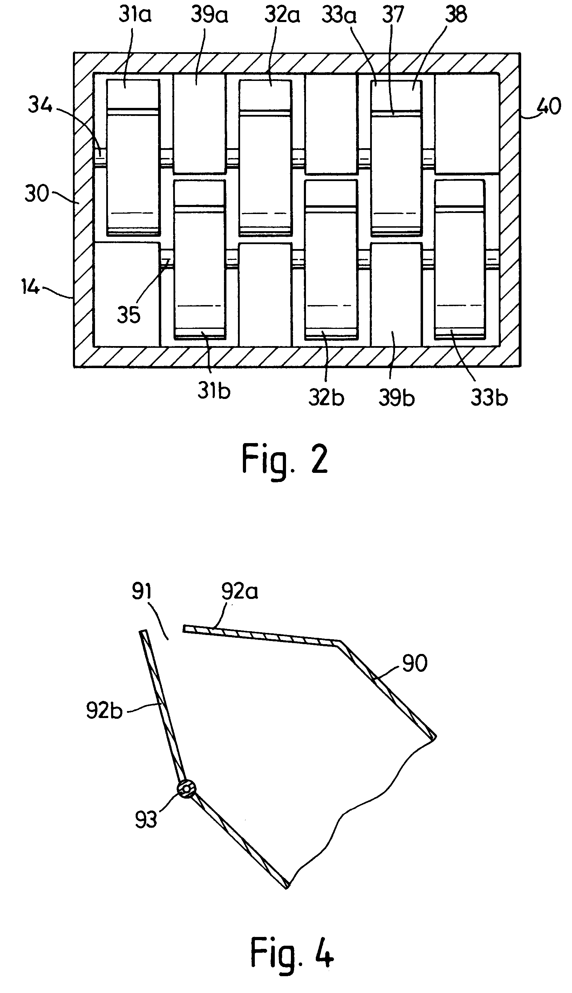

Inlet stage **12** comprises an opening **28** at or near the top of a fragmenting chamber **30** which houses stages **14**, **16**, and **18**. Inlet stage **12** opens down into shredding stage **14** at the upper level of fragmenting chamber 30. As shown in Figure 2, shredding stage **14** comprises multiple pairs of rotating blades **31a,b, 32a,b, 33a,b**. Blades **31a**, **32a**, **33a**, are mounted on shaft **34** and blades **31b**, **32b**, **33b** are mounted on shaft **35** such that blade **31b** is rotatably fitted between blades **31a**, **32a** and so on for all the blades as shown. Each blade **31**, **32**, **33** is a disk **34** having a plurality of hook-shaped teeth **40** about the periphery **41** of disk **39**. Stationary scrapers **39a**, **39b** are fixed to chamber walls **40** opposite blades **31a**, **32a**, **33a** and **31b**, **32b**, **33b** respectively to channel waste into the blades and to prevent waste from accumulating in shredding stage **14**. Shafts **34**, **35** are positioned opposite one another and rotate in a vertical plane which is substantially parallel to the vertical flowpath of the waste. Shredding action is provided by rotating shaft **39** in the opposite direction from shaft **40**.

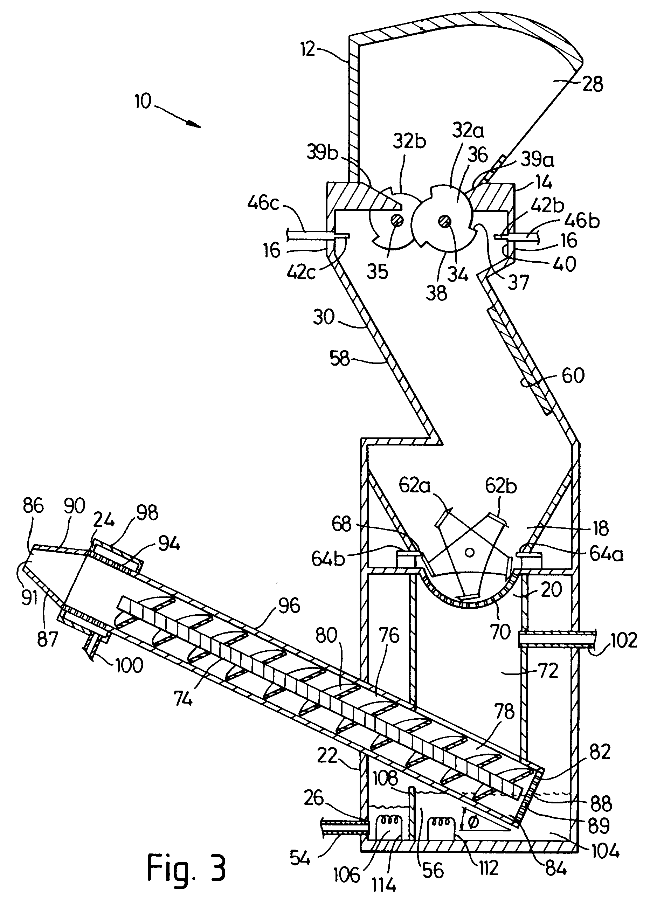

Referring to Figure 3, a wetting stage **16** is provided simultaneous with shredding stage **14**. Wetting stage **16** comprises a plurality of liquid jets **42a,b,c,d** which are mounted in the wall **40** of chamber **30** around the radial periphery of the waste flowpath and adjacent the bottom side of blades **31**, **32**, **33**. A liquid medium feed line **46** is connected to each jet **42**. Thus, as shown in Figure **1**, liquid medium feed lines **46a,b,c,d** are connected to jets **42a,b,c,d** respectively. Feed lines **46** are connected opposite jets **42** to a recycle pump **48** across a liquid distribution manifold **50**. Pump **48** receives liquid medium from a recycle line **54** connected to a liquid medium collection tank **56** of a recycle stage **26**.

If it is desired to remove metals from the waste stream after shredding and wetting, a metal segregating stage **58** may be provided immediately after stages **14**, **16**. Metal segregating stage **58** comprises a magnet **60** which is mounted in the wall **40** of chamber **30**. Magnet **60** contacts the waste as it falls toward granulating stage **18** to segregate the metals therefrom. Access is provided in wall **40** to enable periodic removal of metals from magnet **60**.

Granulating stage **18** is positioned at the lower level of fragmenting chamber **30** and comprises a plurality of travelling blades **62a,b,c,d,e** and stationary blades **64a,b**. Travelling blades **62** are axially mounted on a rotating shaft **66** which in turn is rotatably mounted on chamber wall **40**. Travelling blades **62** have a vertical plane of rotation which is substantially parallel to the vertical flowpath of the waste. Travelling blades **62** are rotatable against stationary blades **66a**, **66b**, each of which is fixably mounted on opposite sides of chamber wall **40** adjacent travelling blades **62**. As travelling blades **62** rotate, travelling blades **62** periodically pass stationary blades **64** to form transient cutting surfaces. Figure 2 shows travelling blade **62b** meeting stationary blade **64b** to form transient cutting surface **68**.

Screening stage **20** is positioned immediately beneath and adjacent granulating stage **18**. Stage **20** comprises a screen **70** stretched cross-sectionally across conduit **72** which connects fragmenting chamber **30** and auger **74**. Auger **74** encloses stages **22** and **24** which are described hereafter. Screen **70** has a mesh size which allows particles at or below a given particle size to pass through while preventing particles having a larger particle size than the given particle size from passing through. Screen **70** preferably has a 1/4 inch mesh size although other mesh sizes are within the purview of the skilled artisan. Screen **70** is positioned to cooperate with travelling blades **62** of granulating stage **18**. As travelling blades **62** rotate, travelling blades **62** periodically pass screen **70** to scoop waste retained on screen **70**. Figure 2 shows travelling blade **62c** meeting screen **70** to return waste retained by screen **70** to transient cutting surface **68**.

Disinfecting stage **22** comprises a disinfectant reaction chamber **76** which is integral with auger **74**. Auger **74** is inclined upward away from auger inlet **78** to enable precise control of the waste residence time in reaction chamber **76** and to facilitate dewatering as described hereafter. The inclination angle of auger **74** is defined as $\phi$. $\phi$ is selected between about 10° and 30° and preferably between about 15° and 25°. Reaction chamber **76** is sufficiently sized to hold the throughput of system **10** for a residence time which enables disinfection of the waste before discharge from system **10**. Auger **74** has a screw **80** extending axially the entire length of auger **74** which is rotatably mounted therein to carry waste from auger inlet **78** to a waste solid discharge port **86** at the upper end **87** of auger **74**.

Dewatering stage **24** is likewise integral with auger **74** and comprises a flow restriction at solid disinfected waste discharge port **86**. A portion of liquid medium exits auger **74** under gravity through port **82** to collection tank **56** in fluid communication with port **82**. A perforated plate **88** is provided at port **82** having a plurality of perforations **89**, each significantly smaller than the mesh size of screen **70**, and preferably about 1/8 inch, to prevent substantial quantities of waste from exiting auger **74** thereat. However, the primary function of port **82** is to enable fluid intrusion into auger **74** as will be shown.

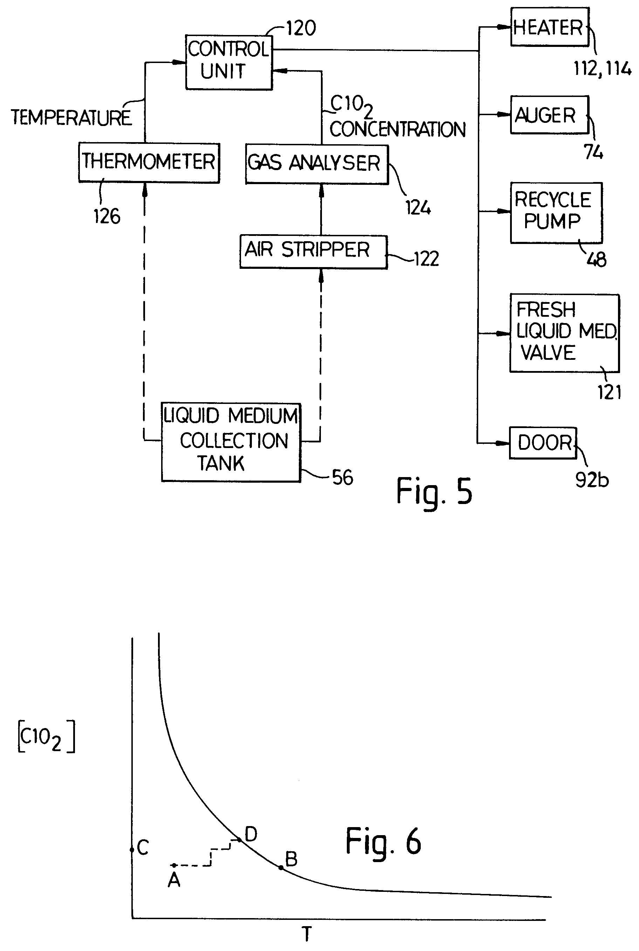

The flow restriction placed at waste discharge port **86** removes the bulk of liquid medium from the waste before it exits system **10**. In one embodiment the constriction is a nozzle **90** having a fixed narrow opening **91** at the end of waste discharge port **86**. In another embodiment, Figure 4 shows an adjustable nozzle comprising a pair of doors **92a**, **92b**, the lower door having a pneumatically biased hinge **93** to render the size of opening **91** pressure responsive. In any case, the restriction applies a compacting force to the disinfected waste before the waste exits the system **10**.

Liquid medium driven from the disinfected waste by the compacting force exits auger **74** through perforations **94** in auger housing **96** adjacent discharge port **86**. Perforations **94** are sized smaller than the waste particle size to restrict the solid waste from the liquid stream. A sleeve **98** around housing **96** at perforations **94** channels the liquid medium into a collection tank return line **100** which is in fluid communication with tank **56** through recycle inlet port **102**.

Collection tank **56** has two chambers **104**, **106** in fluid communication with one another, but separated by a weir **108**. Port **82** of auger **74** is submerged in primary chamber **104** which receives recycle inlet port **102**. Secondary chamber **106** receives the overflow of primary chamber **104** and has a recycle outlet port **110** connected to recycle line **54**. Heater elements **112**, **114** are submerged in primary and secondary chambers **104**, **106** respectively for heating the liquid medium as necessary. Fresh liquid medium may be added to system **10** via a fresh liquid medium line **116** connected to a source (not shown) such as a municipal water line. Line **116** terminates with a jet **118** entering conduit **72**.

Figure 5 is a schematic for process control of system **10** which is provided by automated control unit **120** in electrical communication with auger **74**, heaters **112**, **114**, a valve **121** on fresh liquid line **116**, recycle pump **48** and door **92b**. Control unit **120** accordingly regulates the speed of auger screw **80**, the heat output of heaters **112**, **114**, the fresh liquid medium feed rate of valve **121**, the liquid medium recycle rate of pump **48** and the compaction force applied by door **92b** to the waste at solid waste discharge port **86**. These parameters are regulated in response to the primary input parameters to unit **120** which are the $ClO_2$ concentration and the temperature of the liquid medium in tank **56**. $ClO_2$ concentration data is provided to unit **120** by means of a conventional air stripper **122** in tank **56** and $ClO_2$ gas analyzer **124**. Temperature data is provided to unit **120** from a conventional thermometer **126**.

It is understood that although waste treatment system **10** has been described above in a specific sequence of multiple stages, certain stages may be omitted or reordered within the scope of the present invention as is apparent to one skilled in the art. As such, the present invention is not limited to the above-recited sequence of stages.

METHOD OF OPERATION

With cross-reference to the drawings, operation of system **10** in a continuous mode may be seen. System **10** is particularly suited to the treatment of infectious wastes generated by hospitals and other medical facilities. Such wastes are primarily solid wastes consisting of plastic, paper, fabric, glass, and metal and embody a broad range of medical items including syringes, bottles, tubes, dressings, and the like. "Waste treatment" as the term is used herein constitutes fragmenting of the waste to a relatively small granular particle size and disinfecting the waste to render it substantially innocuous and suitable for ordinary landfilling.

The infectious waste is fed through inlet opening **28** into system **10** in any form. In a preferred embodiment, however, the waste is stored in a sealed compartmentalized plastic bag **128** which is then fed through opening **28** into system **10** in its entirety. Waste bag **128** has a primary compartment **130** containing the infectious waste, and also has other prefilled and sealed compartments **132**, **134** containing disinfectant chemicals or other process additives which are introduced into system **10** via inlet opening **28**. Preferred disinfectant chemicals are dry particulate disinfectant precursors, sodium chlorite and citric acid, which are maintained in isolation in bag **128**, but react in solution as will be shown, to form a disinfectant, i.e., chlorine dioxide. Additives may include dyes, defoamers, or surfactants.

The waste is inserted through inlet opening **28** into the top of fragmenting chamber **30** by an operator. The waste drops under the force of gravity from opening **28** down into opposingly rotating blades **31**, **32**, **33** of shredding stage **14**. Blades **31**, **32**, **33** destroy waste bag **128**, spilling the waste, disinfectant chemicals, and additives into chamber **30** where they are commingled to form a waste mixture. Blades **31**, **32**, **33** also break up the frangible waste to a small particle size. Wetting stage **16** operates simultaneously with stage **14**, whereby jets **42** wet the waste mixture with a stream of a liquid medium. The liquid medium is pumped to jets **42** from recycle line **54** connected to liquid medium collection tank **56**. With efficient operation of dewatering stage **24**, the bulk of liquid medium in system **10** is recycled. The liquid medium is preferably an aqueous liquid, such as fresh tap water and/or recycled process water. The liquid medium may be within a temperature range between about 0°C and 100°C and preferably between about 5°C and 70°C. The liquid medium has more preferably been preheated above ambient temperature to an elevated temperature of at least about 40°C and most preferably at least about 50°C.

The liquid medium uniformly contacts the falling waste mixture to form a wet mash. The mash falls through metal segregating stage **58** where metals are removed and continues falling down into granulating stage **18** where travelling blades **62** and stationary blades **64** break up the already small particle size frangible waste into yet a smaller granular particle size which is preferably slightly less than 1/4 inch. Blades **62**, **64** also fragment any fibrous material which has not been previously fragmented by shredding blades **31**, **32**, **33** to about the same smaller granular particle size as the frangible material. Blades **62**, **64** also more fully mix the mash, thereby dissolving at least a portion of the disinfectant chemicals in the liquid medium to form the chlorine dioxide-containing disinfectant solution. Thus, the solids in the resulting mash of granulating stage **18** are preferably fully wetted by the disinfectant solution and the bulk of the solids preferably have a smaller granular particle size which is slightly less than about 1/4 inch. The liquids content of the mash is typically on the order of about 60% by weight.

Upon exiting granulating stage **18**, the mash drops onto screen **70** which functions in cooperation with the granulating stage **18** to allow the smaller granular particle size waste to fall through it into disinfectant reactor chamber **76** while retaining any waste in granulating stage **18** which has not been sufficiently fragmented. Waste which is retained by screen **70** is scooped up by traveling blades **62**, rotating against screen **70**, and returned to cutting surface **68** for additional particle size reduction until it is sufficiently small to pass through screen **70**.

Inlet port **78** receives the waste mash from screening stage **20** and directs the mash to reactor chamber **76** integral with auger **74**. The disinfectant solution collected in primary chamber **104** contacts the mash initially at lower end **84** of auger **74**. Auger screw **80** turns continuously to withdraw the mash from lower end **84** at angle φ up the auger incline to solid waste discharge port **86** at a controlled rate which allows a sufficient residence time of the mash in reactor chamber **76**. A sufficient residence time is typically on the order of less than about 5 minutes and preferably on the order of about 3 minutes. Auger screw **80** also maintains perforated plate **88** free of waste so that the liquid medium may infiltrate lower end **84** from primary chamber **104** to enhance disinfecting stage **22**. The disinfected and dewatered waste exiting system **10** typically has a liquids content of about 20% by weight in contrast to a liquids content in the mash of about 60% by weight.

The bulk waste volume of the exit waste is on the order of about 15% of the inlet waste. Most of the liquid medium is removed from the waste as the result of compaction caused by fixed nozzle **90** or pressure responsive nozzle **92a,b** positioned at waste discharge port **86**. The liquid medium exits auger **74** through perforations **94** and is collected in tank **56** for recycling to wetting stage **16** via line **54**. The dual-chamber weir arrangement of tank **56** enables collection of fines in primary chamber **104** for periodic removal.

Process control for system **10** is provided by control unit **120**. The decontamination level, i.e., level of kill, attainable in system **10** is a function of several interrelated operating parameters including liquid medium flow parameters and auger and heater operating parameters as shown in Figure 5. Nevertheless, as is shown below, an operational model of system **10** can be developed as a function of a limited number of key parameters, which are level of kill, disinfectant concentration and temperature.

Accordingly, process control can be effected by selecting a desired level of kill, i.e., target kill, and adjusting the disinfectant concentration and disinfectant solution temperature as a function of the operating parameters to meet the preselected target kill. For example, a target kill of 6 decades ($10^6$ organisms/ml) is achieved within about three minutes for a typical infectious medical waste using a chlorine dioxide solution at a concentration of 30 ppm and a temperature of 50°C. In practice, however, the process is controlled by adjusting only temperature while monitoring variations in the disinfectant concentration as a baseline for temperature adjustment. Temperature is selected as the independent variable and disinfectant concentration as the dependent variable for the practical reason that the ability to independently adjust disinfectant concentration is somewhat limited when a fixed amount of precursor is employed, while it is relatively easy to adjust solution temperature via heaters **12, 14**.

The operational model of system **10** recognizes the functional relationship between solution temperature and concentration of the disinfectant, chlorine dioxide, at a given level of kill n. The model is represented by the equation:

$$[ClO_2] = a_n e^{-k_n T} \qquad (1)$$

wherein

$[ClO_2]$ = chlorine dioxide concentration,

T = temperature, and

$a_n$, $k_n$ = empirically determined constants for $kill_n$.

Figure 6 generally depicts the shape of the curve for equation (1). Each point on the curve defines values of $[ClO_2]$ and T at which $kill_n$ can be achieved. Accordingly, process control is more specifically implemented by preselecting the target kill, empirically determining the model constants at the target kill to define a curve, and adjusting the actual values of $[ClO_2]$ and T to lie on the target kill curve.

Figure 6 shows a typical start-up scenario for system **10**. The treatment solution is initially at point A which is inside the required curve for the target kill. Since it is desirable to operate on the curve, automated process control **120** consequently raises the temperature of the solution in tank **56** toward point B which corresponds to the same chlorine dioxide concentration as point A, but at a higher temperature. Raising the temperature of the solution, however, increases the rate of chlorine dioxide formation, thereby increasing the chlorine dioxide concentration of the solution to a value designated by C on the vertical axis. Thus, as point B is approached, control unit **120** calculates that the required temperature on the curve has fallen. The dashed line shows the iterative equilibration procedure followed by control unit **120** whereby an operating point designated by D is ultimately attained. Operation is preferably maintained along or above the locus of points making up the curve which includes point D.

Chlorine dioxide concentration in tank **56** is continuously monitored by means of air stripper **122** and gas analyzer **124** to enable control unit **120** to determine whether the requirements of the disinfectant solution have changed. For example, if a relatively "dirty" waste is fed to system **10**, the amount of $ClO_2$ consumed increases, reducing the $ClO_2$ concentration in the solution. Accordingly, control unit **120** must iteratively increase the temperature of the solution in the manner recited above to return operation of system **10** to the curve. If a relatively "clean" waste is fed to system **10**, the $ClO_2$ concentration increases, correspondingly reducing the temperature requirement. Thus, control unit **120** decreases the temperature of the solution. It is preferable to preselect a target kill exceeding a minimum acceptable level of kill so that adequate decontamination of the waste is achieved even when operation falls somewhat below the curve. It has generally been found that within the presently prescribed temperature range a minimum $ClO_2$ concentration in the treatment solution to achieve an acceptable level of kill is about 10 ppm up to the required concentration and preferably about 12 ppm up to the required concentration.

As noted in the preferred embodiment above, starting quantities of the solid chlorite salt and acid are fixed. As such, they are preferably provided in stoichiometric excess of quantities necessary to produce the required chlorine dioxide concentrations shown on the curve of Figure 6. Thus, adequate concentrations of dissolved precursors will be available in solution for chlorine dioxide production despite the fact that, in most cases, some of the solid precursors do not react, and the additional fact that a significant fraction of the chlorine dioxide is consumed by reaction with the infectious waste constituents or diffuses out of solution. By way of example, a typical relative starting concentration of precursors, solvent and waste which will provide a desired chlorine dioxide concentration, is on the order of 4.6 g/l sodium chlorite/3.3 g/l citric acid/12 kg of solid waste.

While the particular Multi-Stage Infectious Waste Treatment System as herein shown and disclosed in detail is fully capable of obtaining the objects and providing the advantages herein before stated, it is to be understood that it is merely illustrative of the presently preferred embodiments of the invention and that no limitations are intended to the details of construction or design herein shown other than as described in the appended claims.

**Claims**

1. A multi-stage system for treating an infectious waste along a continuous flowpath comprising:

   an inlet stage (12) having an opening (28) sized to receive an infectious waste into the flowpath of said system;
   a shredding stage (14) for reducing the particle size of the waste to a small particle size;
   a disinfecting stage (22) comprising a reactor chamber (76) positioned to receive the wetted granular waste and sized to retain the waste for a selected residence time;
   an auger (74) to transport the waste to a waste discharge port (86); and
   a dewatering stage (24);

   characterized in that

   the shredding stage (14) comprises a plurality of opposingly rotatable blades (31,32,33) in the flowpath of the waste; and further comprising a wetting stage (16) comprising a liquid jet (42) positioned at the flowpath to inject a liquid medium into the waste and to simultaneously wet and mix a disinfectant in the waste material;
   a granulating stage (18) provided beneath the wetting stage (16) for further reducing the particle size of the waste to a smaller granular particle size, said granulating stage (18) having at least one stationary blade (64) and at least one rotatable blade (62) rotatably positionable against said stationary blade (64) to form a cutting surface (68); and wherein the dewatering stage (24) comprises a flow restriction in the waste flowpath, said dewatering stage (24) being in fluid communication with said wetting stage (16) to recycle water from said dewatering stage (24) to said wetting stage (16).

2. A multi-stage system for treating an infectious waste according to claim 1 characterised by further comprising a screening stage (20) having a screen (70) positioned across the flowpath of the waste, said screen (70) sized to enable the smaller granular particle size waste to pass through said screen (70) and to return the small particle size waste to said granulating stage (18).

3. A multi-stage system for treating an infectious waste according to claim 1 or 2 characterised in that the shredding stage (14) comprises first and second blades having planes of rotation substantially parallel to the flowpath of the waste; and wherein the rotatable blade of the granulating stage (18) has a plane of rotation substantially parallel to the flowpath of the waste.

4. A multi-stage system for treating an infectious waste according to any one of claims 1 to 3 characterised in that the auger (74) is inclined upwardly toward the disinfected waste discharge port (86) to facilitate dewatering

5. A multi-stage system for treating an infectious waste according to claim 4 characterised in that said auger (74) has a perforated housing portion (96) proximal and upstream of said discharge port (86), said perforated housing portion having a plurality of perforations (94) smaller than the smaller granular particle size of the waste.

6. A multi-stage system for treating an infectious waste according to claim 4 or 5 characterised in that said auger (74) has an inlet port (78) for receiving the granular waste from said granulating stage (18) and wherein the angle of inclination of said auger (74) is between 10° and 30°.

7. A multi-stage system for treating an infectious waste according to claim 5 characterised by further comprising a liquid medium recycle stage in fluid communication with said plurality of perforations (94), said recycle stage having a collection tank (56) for holding the liquid medium exiting said plurality of perforations (94) and further having a recycle line (100) providing fluid communication between said tank (56) and said wetting stage (16).

8. A multi-stage system for treating an infectious waste according to claim 7 characterised in that the lower end of said auger (74) is submerged and in fluid communication with said collection tank (56).

9. A multi-stage system for treating an infectious waste according to any one of the preceding claims characterised in that said flow restriction is a nozzle (90).

10. A multi-stage system for treating an infectious waste according to any one of the preceding claims characterised by further comprising a metal segregating stage (58) having a means for collecting metals dispersed in the waste passing through said shredding stage (14).

11. A multi-stage system for treating an infectious waste according to any one of claims 7 to 10 characterised in that said recycle stage has a heater (112,114) in heat transfer communication with said collection tank (56).

12. A multi-stage system for treating an infectious waste according to claim 2 characterised in that said screen (70) has a mesh size of about 1\4 inch (0.635 cm).

13. A multi-stage system for treating an infectious waste according to any one of the preceding claims characterised in that said opening (28) is further sized to receive a dry solid disinfectant chemical into the flowpath of said system.

14. A multi-stage system for treating an infectious waste according to claim 9 characterised in that said flow restriction is a nozzle (90,92a,92b) having an adjustable outlet size responsive to pressure against said nozzle (90,92a,92b).

15. A multi-stage system for treating an infectious waste along a continuous flowpath comprising:

an inlet stage (12) having an opening (28) sized to receive an infectious waste into the flowpath of said system;
a shredding stage (14) for reducing the particle size of the waste to a small particle size;
a disinfecting stage (22) comprising a reactor chamber (76) positioned to receive the wetted granular waste and sized to retain the waste for a selected residence time;
an auger (74) to transport the waste to a waste discharge port (86); and
a dewatering stage (24);

characterized in that:

the shredding stage (14) comprises a plurality of opposingly rotatable shredding blades (31a,b;32a,b;33a,b) positioned to receive the waste from the inlet opening (28);
a plurality of stationary shredding blades (39a,39b) positioned opposite said rotatable shredding blades (31a,b;32a,b;33a,b), said stationary shredding blades (39a,39b) being spaced from said rotatable shredding blades (31a,b;32a,b;33a,b) by a selected distance for shredding the waste to a small particle size and further comprising

a wetting stage (16) comprising a plurality of liquid jets (42a,b,c,d) positioned at the flowpath to inject a liquid medium into the waste and to simultaneously wet and mix a disinfectant in the waste material;

a granulating stage (18) provided beneath the wetting stage (16) comprising a plurality of travelling granulating blades (62a,b,c,d,e) positioned to receive the shredded waste from said shredding blades;

a plurality of stationary granulating blades (64a,b), said stationary granulating blades (64a,b) being positioned to form cutting surfaces (68) with said rotatable granulating blades (62a,b,c,d,e) to granulate the shredded waste to a smaller granular particle size free of any strips of waste material not fragmented by the shredding blades;

a screening stage (20) comprising a screen (70) having openings sized to pass the smaller granular particle size waste and to retain larger size particles of waste for continued granulation, said screen (70) being positioned to cooperate with the travelling granulating blades (62); and wherein the dewatering stage (24) comprises

a flow restriction in the waste flowpath and a perforated plate (88) having openings sized to allow liquid to pass through whilst retaining the smaller granular waste, said dewatering stage (24) being in fluid communication with said wetting stage (16) to recycle water from said dewatering stage (24) to said wetting stage (16).

16. A multi-stage system for treating an infectious waste according to claim 15 characterised in that said inlet opening (28) is further provided for receiving a dry solid disinfectant chemical and said shredding, granulating and wetting stage (14,16,18) are further provided for mixing the waste, the chemical, and the liquid medium.

17. A multi-stage system for treating an infectious waste according to claim 16 characterised in that the chemical comprises a disinfectant precursor and said shredding, granulating and wetting stages (14,16,18) are further provided for dissolving at least a portion of the chemical in the liquid medium to form the disinfectant.

18. A multi-stage system for treating an infectious waste according to any one of claims 15 to 17 further comprising a means for heating the liquid medium.

**Patentansprüche**

1. Mehrstufiges Behandlungssystem für infektiösen Müll entlang eines kontinuierlichen Durchflußweges,

mit einer Einlaßstufe (12), die eine zur Aufnahme des infektiösen Mülls im Durchflußweg des Systems ausgebildete Öffnung (28) aufweist,
mit einer Zerkleinerungsstufe (14) zur Reduzierung der Teilchengröße des Mülls auf eine kleinere Teilchengröße,
mit einer Desinfektionsstufe (22), die eine Reaktionskammer (76) für die Ausnahme des befeuchteten granulierten Mülls aufweist, welche groß genug ausgebildet ist, um den Müll für eine gewählte Verweildauer aufzunehmen,
mit einem Schneckenförderer (74) für den Transport des Mülls zum Auslaß (86) für den Müll,
und mit einer Entwässerungsstufe (24),

**dadurch gekennzeichnet**,

daß die Zerkleinerungsstufe (14) eine Mehrzahl gegenläufig drehender Klingen (31, 32, 33) im Durchflußweg des Mülls aufweist,
daß eine Befeuchtungsstufe (16) mit im Durchflußweg angeordneten Flüssigkeitsdüsen (42) vorgesehen ist, um ein flüssiges Medium in den Müll einzuspritzen und gleichzeitig ein Desinfektionsmittel im Müll zu befeuchten und zu mischen,
daß eine Granulierstufe (18) unterhalb der Befeuchtungsstufe (16) zur weiteren Reduzierung dei Teilchengröße des Mülls auf eine kleinere granulare Teilchengröße vorgesehen ist,
wobei die Granulierstufe (18) mindestens eine ortsfeste Klinge (64) und mindestens eine drehbare Klinge (62) aufweist, von denen die drehbare gegenüber der ortsfesten Klinge (64) unter Bildung einer Schnittfläche (60) dreht, und
daß die Entwässerungsstufe (24) eine Durchflußbegrenzung im Durchflußweg des Mülls aufweist, wobei die Entwässerungsstufe (24) durch einen Flüssigkeitsstrom mit der Befeuchtungsstufe (16) kommuniziert, um eine Wiederverwendung des Wassers der Entwässerungsstufe (24) in der Befeuchtungsstufe (16) zu ermöglichen.

2. Behandlungssystem nach Anspruch 1, **dadurch gekennzeichnet**, daß zusätzlich eine Siebstufe (20) mit einem im Durchflußweg angeordneten Sieb (70) vorgesehen ist, wobei das Sieb (70) so groß ausgebildet ist, daß Müll kleinerer Teilchengröße das Sieb (70) passiert und Müll größerer Teilchengröße wieder der Granulierstufe (18) zugeführt wird.

3. Behandlungssystem nach Anspruch 1 oder 2, **dadurch gekennzeichnet**, daß die Zerkeinerungsstufe (14) erste und zweite Klingen mit zum Durchflußweg des Mülls genau parallelen Rotationsebenen aufweist, und daß die Rotationsebene der drehbaren Klinge der Granulierstufe (18) genau parallel zum Durchflußweg das Mülls verläuft.

4. Behandlungssystem nach einem der Ansprüche 1 bis 3, **dadurch gekennzeichnet**, daß der Schneckenförderer (74) zum Auslaß (86) für den desinfizierten Müll hin nach oben geneigt ist, um die Entwässerung zu erleichtern.

5. Behandlungssystem nach Anspruch 4, **dadurch gekennzeichnet**, daß der Schneckenförderer (74) einen perforierten Gehäuseabschnitt (96) in der Nähe flußaufwarts des Auslasses (86) aufweist, wobei des perforierte Gehäuseabschnitt (96) eine Mehrzahl von Öffnungen (94) aufweist, die kleiner als die kleinere granulare Teilchengröße des Mülls sind.

6. Behandlungssystem nach Anspruch 4 oder 5, **dadurch gekennzeichnet**, daß der Schneckenförderer (74) einen Einzug (78) zur Aufnahme des granularen Mülls aus der Granulierstufe (18) aufweist, und daß der Steigungswinkel des Schneckenförderes (74) zwischen 10° und 30° liegt.

7. Behandlungssystem nach Anspruch 5, **dadurch gekennzeichnet**, daß zusätzlich eine Wiederverwertungsstufe für flüssiges Medium vorgesehen ist, die über einen Flüssigkeitsstrom mit den Öffnungen (94) kommuniziert, wobei die Wiederverwertungsstufe einen Sammelbehälter (56) zur Aufnahme des durch die Öffnungen (94) austretenden flüssigen Mediums und zusätzlich eine Rückflußleitung (100) zur Bereitstellung des Kommunikation zwischen dem Sammelbehälter (56) und der Befeuchtungsstufe (16) über einen Flüssigkeitsstrom aufweist.

8. Behandlungssystem nach Anspruch 7, **dadurch gekennzeichnet**, daß das untere Ende des Schneckenförderes (74) eingetaucht ist und über einen Flüssigkeitsstrom mit dein Sammelbehälter (56) kommuniziert.

9. Behandlungsystem nach einem der vorangehenden Ansprüche, **dadurch gekennzeichet**, daß es sich bei dem Durchflußbegrenzer um eine Düse (90) handelt.

10. Behandlungssystem nach einem der vorangehenden Ansprüche, **dadurch gekennzeichnet**, daß zusätzlich eine Metallabscheidungsstufe (58) mit einer Vorrichtung zum Sammeln von im die Zerkleinerungsstufe (14) passierenden Müll enthaltenen Metallen.

11. Behandlungssystem nach einem der Ansprüche 7 bis 10, **dadurch gekennzeichnet**, daß die Wiederverwertungsstufe Heizelemente (112, 114) aufweist, die sich im Wärmeaustauch mit dem Sammelbehälter (56) befinden.

12. Behandlungssystem nach Anspruch 2, **dadurch gekennzeichnet**, daß das Sieb (70) eine Maschenweite von ungefähr 0,635 cm besitzt.

13. Behandlungssystem nach einem der vorangehenden Ansprüchen, **dadurch gekennzeichnet**, daß die Öffnung (28) zusätzlich zur Aufnahme einer trockenen, festen Desinfektionschemikalie in den Durchfluß ausgebildet ist.

14. Behandlungssystem nach Anspruch 9, **dadurch gekennzeichnet**, daß es sich bei dem Durchflußbegrenzer um eine Düse (90, 92a, 92b) handelt, die eine auf Druck gegen die Düse (90, 92a, 92b) reagierende größenverstellbare Auslaßöffnung aufweist.

15. Mehrstufiges Behandlungssystem für infektiösen Müll entlang eines kontinuierlichen Durchflußweges,

mit einer Einlaßstufe (12), die eine zur Aufnahme des infektiösen Mülls im Durchflußweg dem Systems ausgebildete Öffnung (28) aufweist,
mit einer Zerkleinerungsstufe (14) zur Reduzierung der Teilchengröße des Mülls auf eine kleinere Teilchengröße,
mit einer Desinfektionsstufe (22), die eine Reaktionskammer (76) für die Aufnahme des befeuchteten granulierten Mülls aufweist, welche groß genug ausgebildet ist, um den Müll für eine gewählte Verweildauer aufzunehmen,
mit einem Schneckenförderer (74) für den Transport des Mülls zum Auslaß (86) für den Müll,
und mit einer Entwässerungsstufe (24).

**dadurch gekennzeichnet,**

daß die Zerkleinerungsstufe (14) eine Mehrzahl gegenläufig drehender Klingen (31a, b; 32a, b; 33a, b) zur Aufnahme des Mülls aus der Einlaßöffnung (28) aufweist,
daß eine Mehrzahl ortsfester Klingen (39a, 39b) gegenüber den drehbaren Klingen (31a, b; 32a, b; 33a, b) angeordnet sind, wobei die ortsfesten Klingen (39a,39b) einen gewählten Abstand zu den drehbaren Klingen (31a, b; 32a, b; 33a, b) aufweisen, um den Müll auf eine kleine Teichengröße zu zerkleinern,

daß eine Befeuchtungsstufe (16) mit einer Mehrzahl im Durchflußweg angeordneten Flüssigkeitsdüsen (42a, b, c, d) vorgesehen ist, um ein flüssiges Medium in den Müll einzuspritzen und gleichzeitig ein Desinfektionsmittel im Müll zu befeuchten und zu mischen,

daß eine Granulierstufe (18) unterhalb der Befeuchtungsstufe (16) zur weiteren Reduzierung der Teilchengröße des Mülls auf eine kleinere granulare Teilchengröße vorgesehen ist, wobei die Granulierstufe (18) eine Mehrzahl beweglicher granulierender Klingen (62a, b, c, d, e) zur Aufnahmen des zerkleinerten Mülls aus den Klingen für die Zerkleinerung aufweist, daß eine Mehrzahl ortsfester granulierender Klingen (64a, b) vorgesehen ist, wobei die ortsfesten granulierenden Klingen (64a, b) so angeordnet sind, daß sie mit den drehbaren granulierenden Klingen (62a, b, c, d, e) eine Schnittfläche (68) bilden und somit den zerkleinerten Müll frei von nicht durch die zerkleinernden Klingen zerkleinerte Müll-Streifen auf eine kleinere Teilchengröße granulieren,

daß eine Siebstufe (20) mit einem Sieb (70), das Öffnungen aufweist, die es dem Müll kleinerer Teichengröße erlauben zu passieren und den Müll größerer Teilchengröße für weitere Granulierung zurückhalten, vorgesehen ist, wobei das Sieb (70) mit den beweglichen granulierenden Klingen (62) zusammenarbeitet, und

daß die Entwässerungsstufe (24) einen Durchflußbegrenzer im Durchflußweg des Mülls und eine perforierte Platte (88) mit Öffnungen, die so ausgebildet sind, daß Wasser sie passiert, während der Müll kleinerer Granulargröße zurückgehalten wird, aufweist, wobei die Entwässerungsstufe (24) über einen Flüssigkeitsstrom mit der Befeuchtungsstufe (16) kommuniziert, um Wasser aus der Entwässerungsstufe (24) der Befeuchtungsstufe (16) zuzuführen.

16. Behandlungssystem nach Anspruch 15, **dadurch gekennzeichnet**,

daß die Einlaßöffnung (28) zusätzlich der Aufnahme trockener, fester Desinfektionschemikalien dient, und

daß die Zerkleinerungs-, Granulier- und Befeuchtungsstufen (14, 16, 18) zusätzlich der Vermischung von Müll, Chemikalien und flüssigem Medium dienen.

17. Behandlungssystem nach Anspruch 16, **dadurch gekennzeichnet**,

daß die Chemikalien einen desinfizierenden Zwischenstoff beinhalten, und

daß die Zerkleinerungs-, Granulier- und Befeuchtungsstufen (14,16,18) zusätzlich dazu dienen, zumindest einen Teil der Chemikalien im flüssigen Medium zur Bildung eines Desinfektionsmittels zu lösen.

18. Behandlungssystem nach einem der Ansprüche 15 bis 17, **dadurch gekennzeichnet**, daß eine Vorrichtung zum Erhitzen des flüssigen Mediums vorgesehen ist.

## Revendications

1. Système multi-étage pour le traitement de déchets infectieux le long d'une voie d'écoulement continue comprenant :

un étage d'admission (12) ayant une ouverture (28) dimensionnée pour recevoir des déchets infectieux dans la voie d'écoulement dudit système ;
un étage de déchiquetage (14) pour réduire la granulométrie des déchets à une petite granulométrie ;
un étage de désinfection (22) comprenant une chambre de réacteur (76) positionnée pour recevoir les déchets granulaires mouillés et dimensionnée pour retenir les déchets pendant un temps de séjour choisi ;
une tarière (74) pour transporter les déchets vers un orifice de décharge de déchets (86) ; et
un étage d'égouttage (24) ;

caractérisé en ce que

l'étage de déchiquetage (14) comprend une pluralité de lames pouvant tourner en sens contraire (31,32,33) dans la voie d'écoulement des déchets ; et comprenant en outre
un étage de mouillage (16) comprenant un jet de liquide (42) positionné au niveau de la voie d'écoulement pour injecter un milieu liquide dans les déchets et pour simultanément mouiller et mélanger un désinfectant dans les déchets ;
un étage de granulation (18) prévu sous l'étage de mouillage (16) pour réduire encore la granulométrie des déchets à une taille de particule granulaire plus petite, ledit étage de granulation (18) ayant au moins une lame fixe (64) et au moins une lame pouvant tourner (62) pouvant être positionnée de façon à tourner contre ladite lame fixe (64) pour former une surface coupante (68) ; et dans lequel l'étage d'égouttage (24) comprend un dispositif de restriction de flux dans la voie d'écoulement des déchets, ledit étage d'égouttage (24) étant en communication fluidique avec ledit étage de mouillage (16) pour recycler l'eau provenant dudit étage d'égouttage (24) vers ledit étage de mouillage (16).

2. Système multi-étage pour le traitement de déchets infectieux selon la revendication 1, caractérisé en ce qu'il comprend en outre un étage de criblage (20) ayant un crible (70) positionné en travers de la voie d'écoulement des déchets, ledit crible (70) étant dimensionné pour laisser passer les déchets de plus petite taille de particule granulaire à travers ledit crible (70) et pour retourner les déchets de petite granulométrie vers ledit étage de granulation (18).

3. Système multi-étage pour le traitement de déchets infectieux selon la revendication 1 ou 2, caractérisé en ce que l'étage de déchiquetage (14) comprend des première et seconde lames ayant des plans de rotation substantiellement parallèles à la voie d'écoulement des déchets ; et dans lequel la lame pouvant tourner de l'étage de granulation (18) a un plan de rotation substantiellement parallèle à la voie d'écoulement des déchets.

4. Système multi-étage pour le traitement de déchets infectieux selon l'une quelconque des revendications 1 à 3, caractérisé en ce que la tarière (74) est inclinée vers le haut en direction de l'orifice de décharge des déchets désinfectés (86) pour faciliter l'égouttage.

5. Système multi-étage pour le traitement de déchets infectieux selon la revendication 4, caractérisé en ce que ladite tarière (74) a une partie de carter perforée (96) proche et en amont dudit orifice de décharge (86), ladite partie de carter perforée ayant une pluralité de perforations (94) plus petites que la plus petite taille de particule granulaire des déchets.

6. Système multi-étage pour le traitement de déchets infectieux selon la revendication 4 ou 5, caractérisé en ce que ladite tarière (74) a un orifice d'entrée (78) pour recevoir les déchets granulaires provenant dudit étage de granulation (18) et dans lequel l'angle d'inclinaison de ladite tarière (74) est compris entre 10° et 30°.

7. Système multi-étage pour le traitement de déchets infectieux selon la revendication 5, caractérisé en ce qu'il comprend en outre un étage de recyclage du milieu liquide en communication fluidique avec ladite pluralité de perforations (94), ledit étage de recyclage ayant un bassin collecteur (56) pour contenir le milieu liquide sortant de ladite pluralité de perforations (94) et ayant en outre une ligne de recyclage (100) fournissant une communication fluidique entre ledit bassin (56) et ledit étage de mouillage (16).

8. Système multi-étage pour le traitement de déchets infectieux selon la revendication 7, caractérisé en ce que l'extrémité inférieure de ladite tarière (74) est immergée et en communication fluidique avec ledit bassin collecteur (56).

9. Système multi-étage pour le traitement de déchets infectieux selon l'une quelconque des revendications qui précèdent, caractérisé en ce que ledit dispositif de restriction de flux est une buse (90).

10. Système multi-étage pour le traitement de déchets infectieux selon l'une quelconque des revendications qui précèdent, caractérisé en ce qu'il comprend en outre un étage de séparation des métaux (58) ayant des moyens pour recueillir les métaux dispersés dans les déchets passant par ledit étage de déchiquetage (14).

11. Système multi-étage pour le traitement de déchets infectieux selon l'une quelconque des revendications 7 à 10, caractérisé en ce que ledit étage de recyclage a un dispositif de chauffage (112, 114) en communication de transfert thermique avec ledit bassin collecteur (56).

12. Système multi-étage pour le traitement de déchets infectieux selon la revendication 2, caractérisé en ce que ledit crible (70) a une taille de maille d'environ 1/4 pouce (0,635 cm).

13. Système multi-étage pour le traitement de déchets infectieux selon l'une quelconque des revendications qui précèdent, caractérisé en ce que ladite ouverture (28) est en outre dimensionnée pour recevoir un produit chimique désinfectant solide et sec dans la voie d'écoulement dudit système.

14. Système multi-étage pour le traitement de déchets infectieux selon la revendication 9, caractérisé en ce que ledit dispositif de restriction de flux est une buse (90, 92a, 92b) ayant une dimension de sortie réglable en fonction de la pression contre ladite buse (90, 92a, 92b).

15. Système multi-étage pour le traitement de déchets infectieux le long d'une voie d'écoulement continue comprenant :

    un étage d'admission (12) ayant une ouverture (28) dimensionnée pour recevoir des déchets infectieux dans la voie d'écoulement dudit système ;

un étage de déchiquetage (14) pour réduire la granulométrie des déchets à une petite granulométrie ;

un étage de désinfection (22) comprenant une chambre de réacteur (76) positionnée pour recevoir les déchets granulaires mouillés et dimensionnée pour retenir les déchets pendant un temps de séjour choisi ;

une tarière (74) pour transporter les déchets vers un orifice de décharge de déchets (86) ; et un étage d'égouttage (24) ;

caractérisé en ce que :

l'étage de déchiquetage (14) comprend une pluralité de lames déchiqueteuses pouvant tourner en sens contraire (31a,b ; 32a,b ; 33a,b) positionnées pour recevoir les déchets en provenance de l'ouverture d'admission (28) ;

une pluralité de lames déchiqueteuses fixes (39a, 39b) positionnées face auxdites lames déchiqueteuses pouvant tourner (31a,b ; 32a,b ; 33a,b), lesdites lames déchiqueteuses fixes (39a, 39b) étant espacées desdites lames déchiqueteuses pouvant tourner (31a,b ; 32a,b ; 33a,b) d'une distance choisie pour déchiqueter les déchets en une petite taille de particule et comprenant en outre

un étage de mouillage (16) comprenant une pluralité de jets de liquide (42a,b,c,d) positionnés au niveau de la voie d'écoulement pour injecter un milieu liquide dans les déchets et pour simultanément mouiller et mélanger un désinfectant dans les déchets ;

un étage de granulation (18) prévu sous l'étage de mouillage (16) comprenant une pluralité de lames de granulation mobiles (62a,b,c,d,e) positionnées pour recevoir les déchets déchiquetés provenant desdites lames déchiqueteuses ;

une pluralité de lames de granulation fixes (64a,b), lesdites lames de granulation fixes (64a,b) étant positionnées pour former des surfaces coupantes (68) avec lesdites lames de granulation pouvant tourner (62a,b,c,d,e) pour granuler les déchets déchiquetés en une taille de particule granulaire plus petite exempte de toute pièce de déchets non fragmentée par les lames déchiqueteuses ;

un étage de criblage (20) comprenant un crible (70) ayant des ouvertures dimensionnées pour laisser passer les déchets de plus petite taille de particule granulaire et pour retenir les particules de déchets de plus grosse taille pour une granulation complémentaire, ledit crible (70) étant positionné pour coopérer avec les lames de granulation mobiles (62) ; et dans lequel l'étage d'égouttage (24) comprend

un dispositif de restriction de flux dans la voie d'écoulement des déchets et une plaque perforée (88) ayant des ouvertures dimensionnées pour laisser passer le liquide au travers, tout en retenant les plus petits déchets granulaires, ledit étage d'égouttage (24) étant en communication fluidique avec ledit étage de mouillage (16) pour recycler l'eau provenant dudit étage d'égouttage (24) vers ledit étage de mouillage (16).

16. Système multi-étage pour le traitement des déchets infectieux selon la revendication 15, caractérisé en ce que ladite ouverture d'admission (28) est en outre équipée pour recevoir un produit chimique désinfectant solide et sec et lesdits étages de déchiquetage, granulation et mouillage (14, 16, 18) sont en outre prévus pour mélanger les déchets, le produit chimique, et le milieu liquide.

17. Système multi-étage pour le traitement des déchets infectieux selon la revendication 16, caractérisé en ce que le produit chimique comprend un précurseur de désinfectant et lesdits étages de déchiquetage, granulation et mouillage (14, 16, 18) sont en outre prévus pour dissoudre au moins une partie du produit chimique dans le milieu liquide pour former le désinfectant.

18. Système multi-étage pour le traitement de déchets infectieux selon l'une quelconque des revendications 15 à 17, comprenant en outre des moyens pour chauffer le milieu liquide.

Fig. 1

31a   39a   32a   33a   37   38

34

30

14

35

31b    32b   39b   33b

40

# Fig. 2

91   92a

92b

93

90

# Fig. 4

Fig. 3

Fig. 5

Fig. 6